Europäisches Patentamt

European Patent Office

Office européen des brevets

(11) Publication number: **0 191 628 B1**

# EUROPEAN PATENT SPECIFICATION

(45) Date of publication of patent specification: **03.11.93**

(51) Int. Cl.5: **C01F 7/48**, C01F 7/56, A61K 7/38

(21) Application number: **86300916.3**

(22) Date of filing: **11.02.86**

(54) Preparation of basic aluminium halides.

(30) Priority: **13.02.85 GB 8503672**

(43) Date of publication of application:
**20.08.86 Bulletin 86/34**

(45) Publication of the grant of the patent:
**03.11.93 Bulletin 93/44**

(84) Designated Contracting States:
**AT BE CH DE FR GB IT LI NL SE**

(56) References cited:
**AU-B- 150 410**
**GB-A- 2 048 229**
**US-A- 3 904 741**
**US-A- 4 359 456**

(73) Proprietor: **UNILEVER PLC**
**Unilever House**
**Blackfriars**
**P.O. Box 68**
**London EC4P 4BO(GB)**

(84) Designated Contracting States:
**GB**

(73) Proprietor: **UNILEVER N.V.**
**Weena 455**
**NL-3013 AL Rotterdam(NL)**

(84) Designated Contracting States:
**BE CH DE FR IT LI NL SE AT**

(72) Inventor: **Inward, Peter William**
**High Close**
**Wetstone Lane**
**West Kirby**
**Wirral Merseyside L48 7HG(GB)**

(74) Representative: **Ford, Michael Frederick et al**
**MEWBURN ELLIS**
**2 Cursitor Street**
**London EC4A 1BO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3. 10/3.6/3.3. 1)

## Description

This invention relates to the preparation of basic aluminium halides, more particularly to the preparation of a basic aluminium chloride or bromide in the form of a hydrated powder having good antiperspirant activity.

Basic aluminium chlorides and to a lesser extent bromides have been available commercially for many years both in the form of aqueous solutions and in the form of hydrated powders. One well known application of these products is as the active ingredient of antiperspirant products. For such application the basic aluminium chlorides especially are commercially available in varying degrees of basicity, which can be expressed in terms of the aluminium to chlorine ratio which generally ranges from about 2:1 to about 0.9:1. The products available commercially in the form of aqueous solutions generally have an aluminium concentration of about 12% by weight. The powdered forms of the basic aluminium chlorides and bromides are frequently obtained by spray-drying aqueous solutions of these basic halides. These solutions are usually made by dissolving aluminium metal in an aqueous solution of aluminium chloride or bromide at an elevated temperature and such method is described in US-A-2 196 016 (Huehn et al) and AU-A-150,410 (Elliotts & Australian Drug Proprietary Ltd). The amount of aluminium metal dissolved is controlled so as to correspond to a final product having the desired aluminium to halide ratio. Furthermore, the concentration of the aluminium halide solution is chosen so as to give a final product having an aluminium concentration of around 12% by weight which is most advantageous for commercial sale or for conversion to a powdered form, eg. by spray drying such solutions.

In US-A-4 359 456 (Gosling et al), the disclosure of which is incorporated herein by reference, there is described a process for making from commercially available materials a basic aluminium chloride or bromide having enhanced antiperspirant efficacy. The process described by the prior patent involves first forming a solution of the basic aluminium halide having an aluminium concentration of 2.5 to 8.5% by weight. This can be prepared by dissolving in water a commercially available powder of a basic aluminium halide (as in Example 2) or by diluting with water a commercially available solution of a basic aluminium halide (as in Example 9). The solution is then heated at a temperature of 50° to 140°C so as to modify the distribution of the polymeric species within such solution in such manner that the modified basic aluminium halide has at least 20%, preferably at least 25%, of the aluminium contained in the Band III fraction as determined by a chromatographic analytical procedure described in the patent. The products obtained having such high contents of aluminium in the Band III fraction have superior antiperspirant activity. Powdered forms of the improved basic aluminium halides are obtained by drying the heat-treated solutions.

It is now well recognised that the development of powdered forms of basic aluminium halides, especially aluminium chlorhydrate, of high antiperspirant efficacy as first characterised by Gosling et al in US-A-4 359 456 represents a significant advance in the antiperspirant art. Considerable publicity has been given to the value of such materials in a number of publications which have appeared in 1985, for example "Aerosol Age", October 1985, p26 an article entitled "Enhanced Efficacy Actives for Aerosol Antiperspirant Products", "Manufacturing Chemist", November 1985, page 49 an article entitled "Increasing Aerosol Antiperspirant Efficacy", and "Soap/Cosmetics/Chemical Specialities", December 1985, page 44. In, for example, the article in "Aerosol Age" a chromatogram for Chlorhydrol is published having peaks A, B and C which correspond to Bands I, II and III of the Gosling et al patent. In both cases the chromatography was performed on a column packed with Lichrosorb RP-2, the samples contained 2.5% aluminium and were eluted using 0.01N nitric acid as eluent. The materials referred to as "Reach 101" and "Reach 102", respectively, in the article in "Aerosol Age" had significantly enhanced levels of peak C, corresponding to Band III of Gosling et al, and enhanced efficacy. The article in "Aerosol Age" discloses that the materials of enhanced efficacy are prepared by processes which involve the heat treatment of aluminium chlorhydrate solutions bringing about an alteration of the distribution of the molecular species present in the aluminium chlorhydrate solution. This improvement in antiperspirant efficacy which is brought about by the heat treatment of relatively dilute solution of aluminium chlorhydrate is also described in GB-A-2 048 229 (Gillette).

It is an object of the present invention to provide a simplified process for making a powder of a basic aluminium chloride or bromide having a high proportion of the aluminium in the Band III fraction without the need to heat-treat a pre-formed solution of a basic aluminium halide. In the process of the invention a powdered basic aluminium halide having a high content of aluminium in the Band III fraction is prepared directly.

According to the invention there is provided a process of making a basic aluminium halide in powder form having an aluminium:halogen molar ratio of from 1.7 to 2.2 : 1 and having at least 20% of the aluminium contained in the Band III fraction which process comprises the steps of:-

a) dissolving metallic aluminium in an aqueous starting solution of an aluminium compound selected from aluminium chloride and aluminium bromide, said starting solution being held at a temperature of about 50°C to about 105°C, preferably 50°C to 95°C, for a time just long enough to dissolve sufficient aluminium to produce an aqueous solution of a final basic aluminium halide having an aluminium:halide molar ratio in the range 1.7:1 to 2.2:1, the concentration of the aluminium in the starting solution and the amount of aluminium dissolved being such that the aluminium concentration in the solution of the final basic aluminium halide is from 0.8% to 6.75% by weight and said final basic aluminium halide having at least 20% of the aluminium contained in the Band III fraction; and

b) drying the solution of the final basic aluminium halide.

Characterisation of materials containing species differing in size by means of size exclusion chromatography is generally known. The size exclusion chromatographic procedures for characterising the basic aluminium compounds of this invention will now be described.

The analytical procedure is performed on a stainless steel column of dimensions 30 cm high and of 7 mm internal diameter packed with porous silica of nominal particle size 5 $\mu$m and pore size of 60 Å, which silica has been deactivated by silylation to eliminate adsorption and unwanted ionic effects in size exclusion separations. A suitable silica is that available commercially as LiChrosorb RP-2. The silica employed by the Applicants in deriving analytical data given herein had a cumulative undersize particle size distribution by weight of 10% less than 5 microns, 50% less than 6 $\mu$m and 90% less than 7 $\mu$m. (1 Å = $10^{-8}$ cm)

The column is fitted at the bottom with a zero dead volume fitting containing a 2 micron mesh stainless steel bed support. The silica is packed into the column by the high pressure slurry method (see Practical High Performance Liquid Chromatography, Edited by C F Simpson, 1976, Appendix II), using methanol:water (90:10) containing 1% sodium acetate as the packing medium.

After packing, the column is capped with another zero dead volume fitting containing a 2 $\mu$m stainless steel mesh. The packed column is then eluted with 200 ml of methanol at a flow rate of about 10 ml/min, using a high pressure pump, to consolidate the bed and wash out the packing medium. The bed is topped up, if necessary, with a thick slurry of the packing in methanol followed by reconsolidation.

A differential refractive index monitor (eg Waters R401) is used to detect sample fractions as they are eluted. It is linked to a pen recorder to provide a chromatogram and to an integrator (eg. Infotronics CRS 309) which measures the elution times of the fractions and the relative chromatographic band areas. The integrator is required to measure areas of bands not resolved to the baseline by dropping perpendiculars from the lowest point of the valleys separating the bands to the baseline.

The column packing should be tested according to the procedure of Bristow & Knox (Chromatographia, Volume 10, No 6, June 1977, pp 279-89) for reverse phase materials and should generate at least 20,000 plates/metre for the test component phenetole.

To prepare test solutions of the materials for analysis those already in solution are used undiluted unless the aluminium concentration exceeds 2.5% by weight in which case they are diluted with deionized water to give a solution containing 2.5% by weight aluminium and dispersed by treatment in an ultrasonic bath for 2 minutes. Solid materials (eg spray dried powders) are dissolved in deionized water to give a solution containing 2.5% by weight aluminium and dispersed by treatment in an ultrasonic bath for 2 minutes. The solutions prepared in this way are filtered through a 25 mm diameter membrane having a pore size of 0.025 micrometres to give the test solutions. The preparation of a test solution is carried out immediately prior to application of a sample to the column.

A sample of the test solution containing about 2 to 4 micromoles of aluminium is applied to the top of the column by means of a precision micro-litre syringe and a sample injection port. The sample is eluted with a 1 x $10^{-2}$ M aqueous nitric acid solution at a flow rate of 1.0ml/min using a high pressure pump. The temperature of the eluent is about 22-23°C.

Eluted fractions of a test sample are characterised by means of the ratio of their retention times to the retention time of the totally included species. In the case of basic aluminium chlorides the totally included species arises from hydrochloric acid (which is present in solutions of basic aluminium chlorides) as can be shown by comparison of its retention time with that of a sample of hydrochloric acid. Using columns satisfying the above description and employing a standard solution of a basic aluminium chloride prepared as described below, the Applicants have obtained separation into four aluminium-containing fractions having relative retention times within the ranges indicated.

| | Band I | Band II | Band III | Band IV |
| --- | --- | --- | --- | --- |
| Relative Retention Time Range | 0.62-0.70 | 0.71-0.75 | 0.76-0.82 | 0.83-0.97 |

The standard basic aluminium chloride solution is prepared as a solution containing 12.5% by weight aluminium from 19.1 g of aluminium chloride hexahydrate, 10.5 g of 99.9% pure aluminium wire (0.76 mm diameter, cut in approximately 1 cm lengths and degreased by washing in acetone) and 70.4 g of deionised water. The mixture is stirred and heated at 80-90°C under a reflux condenser until all of the aluminium is dissolved. Any traces of insoluble solids are removed by filtration to give a clear solution.

When this material is analysed by the size exclusion chromatographic procedure described herein, there are obtained the following four fractions having typical relative retention times and chromatographic band areas expressed as percentages of the total chromatographic band area representing aluminium-containing material.

|  | Band I | Band II | Band III | Band IV |
|---|---|---|---|---|
| Relative Retention Time | 0.65 | 0.73 | 0.79 | 0.91 |
| Band Area % of total aluminium band area | 39 | 51 | 4 | 6 |

A standard basic aluminium bromide solution was prepared in a manner similar to that as described above for the chloride by employing 29.7 g aluminium bromide hexahydrate, 10.7 g aluminium wire and 59.6 g water. Analysis again gave four fractions having the relative retention times and chromatographic band areas expressed as percentages of the total chromatographic band area representing aluminium-containing material indicated below.

|  | Band I | Band II | Band III | Band IV |
|---|---|---|---|---|
| Relative Retention Time | 0.62 | 0.74 | 0.80 | 0.94 |
| Band Area % of total aluminium band area | 34 | 56 | 7 | 3 |

The standard solutions contained 0% aluminium as polymers greater than 100 Angstroms in effective diameter.

It will be appreciated by those skilled in the art that mechanisms of separation other than the principal mechanism of size exclusion may play a part in this type of chromatography. Examples of the processes would be adsorption effects and hydrodynamic effects. Thus although it is possible for a given column and constant operating conditions to lead to invariable relative retention times, minor variations in particle size range and pore size distribution of the column packing material may lead to slight differences in relative retention times.

Quantitatively, the amount of aluminium in the Band III fraction expressed as a percentage of the total aluminium of the compound under test is readily determined from the area of its band on the chromatogram. This percentage is derived from the expression

$$\% \text{ Aluminium} = (100-A) \times \frac{\text{Area of band corresponding to Band III fraction}}{\text{Sum of the areas of the bands corresponding to the aluminium-containing fractions}}$$

where A is the percentage of the total aluminium which is contained in polymers greater than 100 Å and is determined by the method described hereinafter.

In experiments performed by the Applicants using certain samples of test materials, the complete elution of all the applied aluminium in a sample was checked by direct analysis of another sample of the same volume by plasma emission spectrophotometry. The correlation between band area percentage and aluminium percentage was also verified by direct analysis. The fractions were collected as they emerged from the refractive index monitor and their individual aluminium contents measured also by plasma emission spectrophotometry.

4

For the determination of percentage aluminium in polymeric species having a size greater than 100 Å there may be used a 1.2m x 6.0mm column packed with porous silica beads of particle size 75-125 $\mu$m, and of surface area 350-500 $m^2$/g, and having a maximum pore size of 100 Å. A suitable silica is that available commercially as Porasil A. The use of Porasil silica beads as a column packing in chromatography is referred to in "Gel Permeation Chromatography" by K H Altgelt and L Segal, 1971, pages 16-18. Using an eluent consisting of an aqueous solution containing 0.1 M sodium nitrate and 0.01M nitric acid the silica was conditioned before use by passage of a large sample (eg 0.2 ml of a 5% w/w solution) of an aqueous aluminium chlorhydrate solution heat-treated in a manner known to lead to the formation of polymers of a size greater than 100 Å (see US-A-4 359 456). Samples to be tested were made up in deionized water to approximately 0.2 M aluminium and thoroughly dispersed by treatment (4 minutes) in an ultrasonic bath. About 0.2 ml samples of approximately 0.2 M aluminium solutions were applied to the column by a sample loop system and eluted with the above eluent at a flow rate of 2ml/min. A differential refractive index monitor linked to a pen recorder was used to detect fractions as they were eluted. These fractions were collected and analysed for aluminium by atomic absorption spectrometry. Complete elution of all aluminium applied in each sample was checked by direct analysis of another sample of the same volume. The percentage of the total aluminium which appeared in the fraction eluted at the void volume (sometimes called the exclusion volume) of the column was considered as that deriving from polymeric material of a size greater than 100 Å in effective diameter.

The general reaction of aluminium with an aqueous solution of an aluminium halide is, as indicated, well-known. It is also known that a small quantity of mercury can be included in the reaction mixture to act as a catalyst. Aluminium in the form of, for example, wire, foil, granules or powder may be used.

An essential feature of the process of the invention for making a basic aluminium chloride or bromide in powder form with high antiperspirant activity is the use of proportions of reactants such that when the desired basic aluminium halide is formed, usually at the point when substantially all the aluminium has dissolved, the aluminium concentration of that solution is relatively low and in the range 0.8% to 6.75% by weight, preferably 2.7% to 5.4% by weight. The reaction is most conveniently carried out at atmospheric pressure although elevated pressures, for example, can be used. Within the ranges of operating conditions referred to above the formation of products having a high content of aluminium in the Band III fraction is favoured by the choice of higher final aluminium:halide ratios eg. 1.8 and above and by lower aluminium concentrations of the final solution eg. 2.7% by weight and below. Within the temperature range of 50° to 105°C referred to, temperature has relatively little effect on the proportion of aluminium in the Band III fraction of the final basic aluminium halide. Polymers with an effective diameter greater than 100 Å (10nm) may also be formed but if they are formed the amount of aluminium contained in these polymers is in general low. Amounts of these polymers containing aluminium substantially in excess of 2% of the total aluminium will tend to occur only in reactions conducted at higher temperatures and in the preparation of products having higher aluminium:halide molar ratios in low concentrations. So far as the present invention is concerned the amount of aluminium in polymers of effective diameter greater than 100 Å is not important.

It is also a critical feature of the process of the invention that the solution of the basic aluminium halide formed by dissolution of the metallic aluminium in the aqueous solution of aluminium halide is dried, since the aim of the invention is to provide a powdered form of a basic aluminium halide which has high antiperspirant activity. The drying may be carried out by known techniques and should be carried out without undue delay after the end of the reaction. Suitable methods are referred to in US-A-4 359 456. Spray drying and freeze drying are preferred because these methods give powders which on redissolving in water to the concentration prior to spray drying give solutions having similar characteristics to the original solution.

Preferably the hydrated powder of the final basic aluminium chloride has at least 25% of the aluminium contained in the Band III fraction.

It is also preferred that the powdered basic aluminium halides produced by the process of the invention have an aluminium:halogen molar ratio of 1.9 to 2.1 : 1.

In the direct preparative procedure of the invention, under some conditions products containing a high proportion of the aluminium in the Band III species contain a substantial proportion of this component in the form of a polymer having a characteristic line in the $^{27}$Al NMR spectrum. This line is 62.5 ppm downfield from the resonance of $Al^{3+}(6H_2O)$. This line has been attributed to the presence of a complex ion $[Al_{13}O_4(OH)_{24}(H_2O)_{12}]^{7+}$ by Akitt et al (J.C.S. Dalton Transactions 1972 p604) the structure of which was first established by G Johannson (Acta Chem Scand 1962 Vol 16 p403). This ion has been subsequently referred to as the $Al_{13}O_{40}$ ion by Schonherr et al (Zeitschrift fur Anorganischen und Allgemeinen Chemie, 502, 113-122 (1983)).

5

The quantative determination of the percentage of aluminium present in the $Al_{13}O_{40}$ ion will now be described.

The essence of the measurement is the comparison of the band area of the sharp band from the central Al atom of the $Al_{13}O_{40}$ species [$\delta$ = 62.5 ppm with respect to $Al^{3+}$ hexaaquo = 0] with the area of the aluminate ion band [$\delta$ = 80 ppm]. The aluminate standard (concentration about 0.1M) is contained in a sealed 5 mm NMR tube which is held concentrically inside a 10 mm NMR tube. The annular space between the two tubes is filled with analyte solution. The aluminate standard is freshly made up and calibrated for each series of experiments.

For calibration purposes, this annular space is filled with an aqueous solution containing a known concentration (eg 0.02M) of an aluminium salt, such as Analar aluminium nitrate. From the [27] Al NMR spectrum of this system, the area of the aluminate ion band ($\delta$ = 80 ppm) is compared with that of the $Al^{3+}$ band ($\delta$ = 0 ppm), the effective concentration of aluminium in the aluminate-containing tube being given by the equation:

$$M_S = I_S \times \frac{M_A}{I_A}$$

where

$M_s$     is the effective molar concentration of aluminium in the aluminate solution

$M_A$     is the molar concentration of aluminium in the $Al(NO_3)_3$ solution

$I_s$     is the area of the aluminate band

$I_A$     is the area of the $Al^{3+}$ band from $Al(NO_3)_3$.

Thus $M_s$ is the 'calibration factor' of the sealed tube of aluminate solution, and the use of this tube, as indicated above, with subsequent analyte solutions of unknown composition will allow the amount of aluminium associated with any particular sharp spectral band from the analyte solution to be determined.

Measurement of the central aluminium band of $Al_{13}O_{40}$ species determines only one thirteenth of the aluminium content (the other 12 aluminium atoms produce a resonance band which is too broad to be measured accurately). Hence the total amount of aluminium present in the $Al_{13}O_{40}$ species is obtained by multiplying the area of the central band ($\delta$ = 62.5 ppm) by 13.

The concentration of aluminium thus calculated to be present in the $Al_{13}O_{40}$ species is expressed as a percentage of the total aluminium concentration of the analyte solution which if unknown may be determined eg. by atomic absorption spectrometry.

All NMR measurements were carried out using a Bruker W.M.360 spectrometer with a probe free from background aluminium signal. Sample tubes were made from quartz which is also free from background aluminium signal. The aluminium concentration of the analyte solutions whose $Al_{13}O_{40}$ concentration was to be determined was in the range 0.3M to 1.0M.

In the process of the present invention the production of the $Al_{13}O_{40}$ ion is strongly influenced by the temperature of the reaction with the lower temperatures favouring production of this particular aluminium-containing species. Since the $Al_{13}O_{40}$ species constitutes part of the species present in the Band III fraction, those conditions which favour the latter species also, of course, require to be employed. A further factor favouring production of the $Al_{13}O_{40}$ species is the use of shorter reaction times such as can result from the use of more reactive forms of aluminium, such as powders of high surface area, and use of catalysts.

In one embodiment of the process of the invention at least 20% of the aluminium of the final basic aluminium compound formed in step (a) is in the form of $Al_{13}O_{40}$ species. In a particular form of the process the hydrated powder of the final basic aluminium halide contains at least 25% of the aluminium in the Band III fraction and at least 20% of the aluminium in $Al_{13}O_{40}$ species.

It is believed that spray-dried and freeze-dried powders of basic aluminium halides having an aluminium:halide molar ratio of 1.7:1 to 2.2:1 and having at least 20% of the aluminium present in $Al_{13}O_{40}$ species are novel.

Such powders have the advantage that in addition to their high antiperspirancy they can be used to prepare more concentrated aqueous solutions of basic aluminium halides with a more stable Band III fraction because the $Al_{13}O_{40}$ species is relatively stable in concentrated aqueous solution. Thus aqueous antiperspirant products prepared from such powders would retain their antiperspirant efficacy for longer periods. Conventional products based on aqueous solutions of basic aluminium halides typically have an aluminium concentration of from about 3.5% to about 6.5% by weight in the aqueous phase.

At the present time commercially available basic aluminium halides have relatively low proportions of aluminium present in $Al_{13}O_{40}$ species. Examples of some materials that have been analysed by the applicant are aluminium chlorhydrate powders present in two commercially available aerosol antiperspirant products each claiming superior efficacy and which powders had the following characteristics

|  | %Al in Band III fraction | %Al in $Al_{13}O_{40}$ species | %Al in polymers > 100 Angstroms |
|---|---|---|---|
| 1st product | 42 | 4 | 0 |
| 2nd product | 46 | 2 | 12 |

Also analysed was a sample of aluminium chlorhydrate designated "Reach 101" and supplied by the Reheis Chemical Company for which superior antiperspirant efficacy is also claimed (see "Aerosol Age", October 1985, p26). This had 54% aluminium in the Band III fraction, 2% aluminium in $Al_{13}O_{40}$ species and 13% aluminium in polymers greater than 100 Angstroms. Apart from these recently introduced products, three other longer established conventional basic aluminium chlorides have been analysed for their $Al_{13}O_{40}$ contents and similar values obtained, the highest being about 3% aluminium.

The powdered products obtained by the process of the invention can be used in the formulation of a variety of antiperspirant products including products comprising an aqueous or aqueous alcoholic solution of the basic aluminium halide. However, they are especially suitable for use in formulations comprising an antiperspirant agent suspended in a carrier, which may be a liquid carrier or a solid carrier. Such product types are well-known and may be in the form of an aerosol, lotion or stick product. Such suspension type products wherein the powdered basic aluminium halide has an aluminium:halide molar ratio of 1.7 to 2.2:1 and has at least 20% of the aluminium contained in $Al_{13}O_{40}$ species are believed to be novel.

The following detailed Examples 1 to 15 illustrate the invention. Examples A to E are given for comparative purposes. Analytical data relating to Examples 5 to 15 and A to E are summarised in Table I. Examples 16 to 18 refer to antiperspirant compositions utilising powders of basic aluminium halides made according to the invention.

EXAMPLE 1

Aluminium chloride hexahydrate (3.32g) was dissolved in water (244.8g). This solution was placed in a conical flask fitted with a thermometer and air condenser. Degreased aluminium foil (1.855g) and a magnetic stirrer bar were added and the temperature raised to 75°C with stirring on a magnetic stirrer-hotplate. The reaction mixture was stirred and maintained at 75°C ± 5°C until all the aluminium had dissolved. The aluminium concentration of this solution was about 0.9% by weight. A sample was taken for analysis, and the remainder was frozen in an alcohol bath at -55°C prior to freeze drying using a Lyolab BII freeze-dryer (Life Science Labs Ltd). The solution contained 97% of the aluminium in the Band III fraction falling to 93% on freeze drying. The % $Al_{13}$ value was 30%.

EXAMPLE 2

Aluminium chloride hexahydrate (40.24g) was dissolved in water (937.3g). The solution was placed in a conical flask fitted with a thermometer and an air condenser. A magnetic stirrer bar was added and the temperature raised to 90°C with stirring on a magnetic stirrer-hotplate. Aluminium powder (22.48g) was added in portions. The first portion (about one tenth) was added during the initial warm-up stage, and the remaining portions (three tenths, and the balance of six tenths) were added shortly before the previous addition of aluminium had dissolved. The mixture was stirred and maintained at 90° ± 5°C until all the aluminium had dissolved. This solution was then filtered and pooled with the product from two other identical reactions made at the same time. The pooled solution had an aluminium:chlorine ratio of 2.03 and an aluminium concentration of 2.8% by weight. Drying was carried out using a Niro Mobile Minor spray drier, adjusted to the following operational conditions.

| Inlet temperature | 290°C |
| Outlet temperature | 90°C |
| Input flowrate | 60 ml/min |

The powder was further dried in a fan oven at 105°C for 2 hours and sieved to remove particles greater than 74 microns. The powder had an aluminium:chlorine ratio of 2.12 and a water content of 12.3% by weight.

The solution prior to spray drying contained 64% of the aluminium in the Band III fraction and 36% of the aluminium in the $Al_{13}$ species and for the powder the corresponding values were 67% and 32% respectively. For the powder the amount of aluminium contained in polymers having a size greater than 100 Å was 1%.

EXAMPLE 3

Aluminium chloride hexahydrate (50.30g) was dissolved in water (1171.6g). The solution was placed in a conical flask fitted with a thermometer and an air condenser. Aluminium powder (28.1g) and a magnetic stirrer bar was added, and the temperature raised to 55°C with stirring on a magnetic stirrer-hotplate. The mixture was stirred and maintained at 55 ± 5°C until nearly all the aluminium had dissolved. The mixture was then filtered and pooled with the product from two other identical reactions made at the same time. The pooled solution had an aluminium:chlorine ratio of 1.96 and an aluminium concentration of 2.7% by weight. Drying was carried out using a Niro Mobile Minor spray drier, adjusted to the following operational conditions.

| Inlet temperature | 298 ± 2°C |
| Outlet temperature | 100° |
| Input flowrate | 58 ml/min |

The powder was further dried in a fan oven at 105°C for 2 hr and sieved to remove particles greater than 74 $\mu$m.

The solution prior to spray drying contained 86% of the aluminium in the Band III fraction and 74% of the aluminium in the $Al_{13}$ species and for the powder the corresponding values were 87% and 67% respectively. The powder had an aluminium:chlorine ratio of 2.01 and a water content of 13.0%.

EXAMPLE 4

Aluminium chloride hexahydrate (50.3g) was dissolved in water (1171.6g). The solution was placed in a conical flask fitted with a thermometer and an air condenser. Aluminium powder (28.1g) and a magnetic stirrer bar was added, and the temperature raised to 55°C with stirring on a magnetic stirrer-hotplate. The mixture was stirred and maintained at 55 ± 5°C until the aluminium had substantially dissolved. The mixture was then filtered and pooled with the product from two other identical reactions made at the same time. A total of 4.5g unreacted aluminium was recovered by the filtrations. The pooled solution had an aluminium:chlorine ratio of 1.91 and an aluminium concentration of 2.6% by weight.

Drying was carried out using a Niro Mobile Minor spray-drier adjusted to the following operational conditions

| Inlet temperature | 300°C |
| Outlet temperature | 105°C |
| Input flowrate | 54 ml/min |

The solution prior to spray drying contained 89% of the aluminium in the Band III fraction and 76% of the aluminium in the $Al_{13}$ species and for the powder the corresponding values were 91% and 68%.

EXAMPLE 5

Aluminium chloride hexahydrate (20.12g) was dissolved in water (218.64g). This solution was placed in a conical flask fitted with a thermometer and air-condenser. A magnetic stirrer-bar and a drop of mercury were added and the temperature raised to 55°C with stirring on a magnetic stirrer-hotplate. Degreased aluminium foil (11.24g) was added in ten approximately equal portions, each portion being added as the dissolution of the previous one was nearly complete. The reaction mixture was stirred and maintained at 55°C ± 5° until all the aluminium had dissolved. The mixture was then filtered and a sample taken for analysis.

The basic aluminium compound in powder form is obtained by freeze drying or spray drying the solution of the basic aluminium compound.

EXAMPLES 6 TO 10

In Examples 6 to 10, the reaction was carried out as described for Example 5, with variations in the temperature of reaction, final aluminium concentration and aluminium/chlorine atomic ratio as given in Table I. Any evaporation losses were made good by the addition of deionised water.

The basic aluminium compound in powder form is obtained by freeze drying or spray drying the solution of the basic aluminium compound.

EXAMPLE 11

Aluminium chloride hexahydrate (20.12g) was dissolved in water (218.64g). This solution was placed in a conical flask fitted with a thermometer and air-condenser. A magnetic stirrer bar was added and the temperature raised to 90°C with stirring on a magnetic stirrer-hotplate. Degreased aluminium foil (11.24g) was added in 10 approximately equal portions, each portion being added as the dissolution of the previous one was nearly complete. The reaction mixture was stirred and maintained at 90°C ± 5° throughout the reaction until all the aluminium had dissolved. Evaporation losses were made good by the addition of deionised water and the solution filtered and a sample taken for analysis.

The basic aluminium compound in powder form is obtained by freeze drying or spray drying the solution of the basic aluminium compound.

EXAMPLES 12 AND 13

For these Examples the reactions were carried out as described for Example 11, with variations in the temperature of reaction, final aluminium concentration and aluminium: chlorine atomic ratio as given in Table I.

The basic aluminium compound in powder form is obtained by freeze drying or spray drying the solution of the basic aluminium compound.

EXAMPLES 14 AND 15

These Examples were carried out in a manner similar to that for Example 12 except that aluminium powder was used instead of aluminium foil. The details are given in Table I.

The basic aluminium compound in powder form is obtained by freeze drying or spray drying the solution of the basic aluminium compound.

Example A

Aluminium chloride hexahydrate (45.65g) was dissolved in water (204.35g). This solution was placed in a conical flask fitted with a thermometer and air-condenser. A magnetic stirrer bar and a drop of mercury were added and the temperature raised to 90°C on a magnetic stirrer hotplate. Degreased aluminium foil (25.53g) was added in approximately two equal portions, each portion being added as the dissolution of the previous one was nearly complete. The reaction mixture was stirred and maintained at 90°C ± 5° until all the aluminium had dissolved. Evaporation losses were made good by the addition of deionised water, the solution filtered and a sample taken for analysis.

Examples B to D

In these comparative Examples the reaction was carried out as in comparative Example A with variations in temperature of reaction, final aluminium concentration and Al:Cl atomic ratio as given in Table 1.

Example E

Aluminium chloride hexahydrate (45.27g) was dissolved in water (179.4g). This solution was placed in a conical flask fitted with a thermometer and air-condenser. A magnetic stirrer bar was added and the temperature raised to 90°C on a magnetic stirrer hotplate. Degreased aluminium foil (25.29g) was added in approximately ten equal portions, each successive portion being added as the dissolution of the previous one was nearly complete. The reaction mixture was stirred and maintained at 90°C ± 5° until all of the aluminium had dissolved. Evaporation losses were made good by the addition of deionised water, the solution filtered and a sample taken for analysis.

EP 0 191 628 B1

<u>TABLE I</u>

| Example | Wt(g) AlCl$_3$.6H$_2$O | Wt(g)Al | Wt(g)Water | Final Al concentration (%w/w) | Al/Cl molar ratio | Reaction Temp.(°C) | %Al in Band III (Al$_{13}$ polymer)* |
|---|---|---|---|---|---|---|---|
| 5 | 20.12 | 11.24 | 218.64 | 5.4 | 2.0 | 55 | 34 (15) |
| 6 | 20.12 | 11.24 | 218.64 | 5.4 | 2.0 | 75 | 35 (11) |
| 7 | 20.12 | 11.24 | 218.64 | 5.4 | 2.0 | 90 | 29 ( 3) |
| 8 | 10.06 | 5.62 | 234.30 | 2.7 | 2.0 | 55 | 75 (38) |
| 9 | 10.06 | 5.62 | 234.3 | 2.7 | 2.0 | 75 | 77 |
| 10 | 10.06 | 5.62 | 234.3 | 2.7 | 2.0 | 90 | 63 ( 9) |
| 11 | 20.12 | 11.24 | 218.64 | 5.4 | 2.0 | 90 | 27 |
| 12 | 10.06 | 5.62 | 234.3 | 2.7 | 2.0 | 90 | 49 |
| 13 | 11.17 | 5.50 | 233.3 | 2.7 | 1.8 | 55 | 65 |
| 14 | 10.06 | 5.62 | 234.3 | 2.7 | 2.0 | 90 | 68 |
| 15 | 10.06 | 5.62 | 234.3 | 2.7 | 2.0 | 75 | 78 |
| A | 45.65 | 25.53 | 204.35 | 11.1 | 2.0 | 90 | 3 ( 0) |
| B | 45.27 | 25.29 | 179.4 | 12.2 | 2.0 | 75 | 4 ( 0) |
| C | 45.27 | 25.29 | 179.4 | 12.2 | 2.0 | 55 | 8 ( 1) |
| D | 20.12 | 4.49 | 225.4 | 2.7 | 1.0 | 55 | 13 |
| E | 45.27 | 25.29 | 179.4 | 12.2 | 2.0 | 90 | 7 |

*the values in parenthesis represent the %Al in Al$_{13}$ polymer as measured by $^{27}$Al nmr.

The aqueous solutions of the basic aluminium chlorides of Table I contained substantially no polymeric species having a size greater than 100 Å.

## EXAMPLE 16

The spray dried powder of Example 2 was incorporated into a suspension roll-on product of the following formulation.

| Ingredient | % (w/w) |
|---|---|
| Powder of Example 2 | 12.5 |
| Bentone 38 | 5.0 |
| Volatile silicone* | 79.0 |
| Ethanol | 2.0 |
| Water | 0.5 |
| Perfume | 1.0 |

\* Union Carbide Y7207

This product was compared in a standard hot-room test against a similar product containing a powder obtained in accordance with US-A-4 359 456 by heat-treating a commercially available aluminium chlorhydrate and spray drying the solution to obtain a powder having 46% aluminium in the Band III fraction and 2% aluminium in $Al_{13}O_{40}$ species. This powder had an aluminium:chlorine ratio of 2.05 and a water content of 17.8%.

In the standard hot-room test for the measurement of antiperspirancy, each of a panel of 32 women were treated with one of the above products on the left axilla and the other on the right. Each product was applied to an equal number of right and left axillae. After three daily applications of the appropriate product pairs, panellists were subjected to a 20 minute sweat collection period at $40° \pm 2°C$ and $40\% \pm 5\%$ relative humidity after a 40 minute warm-up period under the same conditions. Sweat was collected in pre-weighed absorbent pads placed in the axilla and weighed. The log transformed sweat weights were then subjected to a t-test for statistical significance by an internationally accepted method and the mean % reduction in sweating calculated from

$$\left[1 - \frac{T}{C}\right] \times 100$$

where one of the products is arbitrarily designated the test product and the other the control. T represents the geometric mean sweat weight from the axillae treated with the test product and C is the geometric mean sweat weight from the axillae treated with the control product.

When subjected to this test, axillae treated with the product containing the powder prepared according to US-A-4 359 456 produced 3% more sweat than those treated with the product of this Example. The difference was not statistically significant at the 90% level.

## EXAMPLE 17

A roll-on antiperspirant product was prepared as described in Example 16 save that the powder of Example 3 was used in place of the powder of Example 2.

This product was compared in the standard hot-room test described in Example 16 against the product referred to in Example 16 containing the powder obtained in accordance with US-A-4 359 456.

In the standard hot-room test, the axillae treated with the product of this Example produced 1.5% more sweat than those treated with the product containing the powder of US-A-4 359 456. This difference did not approach statistical significance at the 90% level.

## EXAMPLE 18

A roll-on antiperspirant preparation was prepared as described in Example 16 save that the powder of Example 4 was used in placed of the powder of Example 2.

Where references are made herein to the percentage of aluminium contained in the Band III fraction of a basic aluminium compound it will be understood that this has the same meaning as the Band III percent

EP 0 191 628 B1

aluminium value as used in US-A-4 359 456.

## Claims

1. Process of making a basic aluminium halide in hydrated powder form having an aluminium:halogen molar ratio of from 1.7 to 2.2 : 1 and having at least 20% of the aluminium contained in the Band III fraction as determined by the size exclusion chromatography test herein before described which process comprises the steps of:-

   a) dissolving metallic aluminium in an aqueous starting solution of an aluminium compound selected from aluminium chloride and aluminium bromide, said starting solution being held at a temperature of about 50°C to about 105°C for a time just long enough to dissolve sufficient aluminium to produce an aqueous solution of a final basic aluminium halide having an aluminium:halide molar ratio in the range 1.7:1 to 2.2:1, the concentration of the aluminium in the starting solution and the amount of aluminium dissolved being such that the aluminium concentration in the solution of the final basic aluminium halide is from 0.8% to 6.75% by weight and said final basic aluminium halide having at least 20% of the aluminium contained in the Band III fraction; and

   b) drying the solution of the final basic aluminium halide

2. Process as claimed in Claim 1 wherein said starting solution is an aqueous solution of aluminium chloride.

3. Process as claimed in Claim 1 or Claim 2 wherein the hydrated powder of the final basic aluminium halide has at least 25% of the aluminium contained in the Band III fraction.

4. Process as claimed in any of Claims 1 to 3 wherein the final basic aluminium halide powder has an aluminium:halide molar ratio of 1.9 to 2.1:1.

5. Process as claimed in any of Claims 1 to 4 wherein the drying of the solution of the final basic aluminium halide in step (b) is carried out by spray drying or freeze drying.

6. Process as claimed in any of Claims 1 to 5 wherein at least 20% of the aluminium of the final basic aluminium compound formed in step (a) is formed in the form of $Al_{13}O_{40}$ species.

7. Process as claimed in any of Claims 1 to 6 wherein the hydrated powder of the basic aluminium halide contains at least 25% of the aluminium formed in the Band III fraction and at least 20% of the aluminium formed in $Al_{13}O_{40}$ species.

8. A spray-dried or freeze dried hydrated powder of a basic aluminium chloride having an aluminium:chlorine molar ratio of 1.7:1 to 2.2:1 and having at least 25% of the aluminium in $Al_{13}O_{40}$ species.

9. An antiperspirant composition comprising a powdered basic aluminium halide suspended in a carrier medium wherein the basic aluminium halide has an aluminium:halide molar ratio of 1.7 to 2.2:1 and has at least 20% of the aluminium contained in the form of $Al_{13}O_{40}$ species.

## Patentansprüche

1. Verfahren zur Herstellung eines basischen Aluminiumhalogenids in hydratisierter Pulverform, das ein molares Verhältnis von Aluminium : Halogen im Bereich von 1,7 bis 2,2 : 1 aufweist und das zumindest 20 % des in der Bande III-Fraktion enthaltenen Aluminiums enthält, wie es durch den vorstehend beschriebenen Größen-Ausschluß-Chromatographietest bestimmt wird, welches verfahren die nachfolgenden Stufen umfaßt:

   (a) Auflösen von metallischem Aluminium in einer wässerigen Ausgangslösung einer Aluminiumverbindung, ausgewählt aus Aluminiumchlorid und Aluminiumbromid, wobei die Ausgangslösung auf einer Temperatur von etwa 50°C bis etwa 105°C, für einen Zeitraum gehalten wird, gerade lange genug, um ausreichend Aluminium zur Bildung einer wässerigen Lösung eines endgültigen basischen Aluminiumhalogenids mit einem molaren Verhältnis von Aluminium : Halogenid in dem Bereich von 1,7 : 1 bis 2,2 : 1 aufzulösen, wobei die Konzentration des Aluminiums in der Ausgangslösung und die Menge des aufgelösten Aluminiums derart sind, daß die Aluminiumkonzentration in der Lösung des endgültigen basischen Aluminiumhalogenids von 0,8 bis 6,75 Gewichtsprozent beträgt, und das erwähnte endgültige basische Aluminiumhalogenid zumindest 20 % des in der Bande III-Fraktion enthaltenen Aluminiums enthält; und
(b) Trocknen der Lösung des endgültigen basischen Aluminiumhalogenids.

2. Verfahren nach Anspruch 1, **dadurch gekennzeichnet,** daß die Ausgangslösung eine wässerige Lösung von Aluminiumchlorid ist.

3. Verfahren nach einem der Ansprüche 1 oder 2, **dadurch gekennzeichnet,** daß das hydratisierte Pulver des endgültigen basischen Aluminiumhalogenids zumindest 25 % des in der Bande III-Fraktion enthaltenen Aluminiums enthält.

4. Verfahren nach einem der Ansprüche 1 bis 3, **dadurch gekennzeichnet,** daß das endgültige basische Aluminiumhalogenid-Pulver ein molares Verhältnis von Aluminium : Halogenid von 1,9 bis 2,1 : 1 aufweist.

5. Verfahren nach einem der Ansprüche 1 bis 4, **dadurch gekennzeichnet,** daß das Trocknen der Lösung des endgültigen basischen Aluminiumhalogenids in Stufe (b) durch Sprühtrocknen oder Gefriertrocknen durchgeführt wird.

6. Verfahren nach einem der Ansprüche 1 bis 5, **dadurch gekennzeichnet,** daß zumindest 20 % des Aluminiums der in Stufe (a) gebildeten endgültigen basischen Aluminiumverbindung in der Form von $Al_{13}O_{40}$-Arten gebildet sind.

7. Verfahren nach einem der Ansprüche 1 bis 6, **dadurch gekennzeichnet,** daß das hydratisierte Pulver des basischen Aluminiumhalogenids zumindest 25 % des in der Bande III-Fraktion gebildeten Aluminiums und zumindest 20 % des in $Al_{13}O_{40}$-Arten gebildeten Aluminiums enthält.

8. Ein sprühgetrocknetes oder gefriergetrocknetes hydratisiertes Pulver eines basischen Aluminiumchlorids mit einem molaren Verhältnis von Aluminium : Chlor von 1,7 : 1 bis 2,2 : 1 und das zumindest 25 % des Aluminiums in $Al_{13}O_{40}$-Arten aufweist.

9. Schweißhemmende Zusammensetzung, **dadurch gekennzeichnet,** daß sie ein pulverisiertes basisches Aluminiumhalogenid, suspendiert in einem Trägermedium, enthält, worin das basische Aluminiumhalogenid ein molares Verhältnis von Aluminium : Halogenid von 1,7 bis 2,2 : 1 aufweist und das zumindest 20 % des in der Form von $Al_{13}O_{40}$-Arten enthaltenen Aluminiums enthält.

**Revendications**

1. Procédé de préparation d'un halogénure basique d'aluminium en poudre hydratée ayant un rapport molaire aluminium:halogène compris entre 1,7:1 et 2,2:1 et ayant au moins 20% de l'aluminium contenu dans la fraction de la Bande III qu'on détermine par chromatographie d'exclusion de dimensions, test qui a déjà été décrit, procédé qui consiste :
a) à dissoudre l'aluminium métallique dans une solution aqueuse de départ d'un composé d'aluminium qui est un chlorure ou un bromure d'aluminium, ladite solution de départ étant maintenue à une température d'environ 50 à 105°C, pendant une durée tout juste suffisante pour dissoudre une quantité suffisante d'aluminium pour produire une solutio aqueuse d'un halogénure basique final d'aluminium présentant un rapport molaire aluminium/halogénure compris entre 1,7:1 et 2,2:1, la concentration de l'aluminium dans la solution de départ et la quantité d'aluminium dissoute étant telles que la concentration d'aluminium dans la solution de l'halogénure basique final d'aluminium est de 0,8 à 6,75% en poids et ledit halogénure basique final d'aluminium présente au moins 20% de l'aluminium contenu dans la fraction de la Bande III ; et
b) à sécher la solution de l'halogénure basique final de l'aluminium.

2. Procédé selon la revendication 1, dans lequel ladite solution de départ est une solution aqueuse de chlorure d'aluminium.

3. Procédé selon la revendication 1 ou 2, dans lequel la poudre hydratée de l'halogénure basique final de l'aluminium comprend au moins 25% de l'aluminium contenu dans la fraction de la Bande III.

4. Procédé selon l'une quelconque des revendications 1 à 3, dans lequel le rapport molaire aluminium : halogénure dans la poudre d'halogénure d'aluminium final basique est compris entre 1,9:1 et 2,1:1.

5. Procédé selon l'une quelconque des revendications 1 à 4, dans lequel on effectue le séchage de la solution de l'halogénure basique final de l'aluminium au stade (b) par séchage par pulvérisation ou par lyophilisation.

6. Procédé selon l'une quelconque des revendications 1 à 5, dans lequel au moins 20% de l'aluminium du composé basique final d'aluminium du stade (a) sont formés par le composé $Al_{13}O_{40}$.

7. Procédé selon l'une quelconque des revendications 1 à 6, dans lequel la poudre hydratée de l'halogénure basique d'aluminium contient au moins 25% de l'aluminium formé dans la fraction de Bande III et au moins 20% de l'aluminium formé dans le composé $Al_{13}O_{40}$.

8. Poudre hydratée séchée par pulvérisation ou lyophilisée d'un chlorure basique d'aluminium ayant un rapport molaire aluminium : chlore compris entre 1,7:1 et 2,2:1 et ayant 25% de l'aluminium au moins dans le composé $Al_{13}O_{40}$.

9. Composition antiperspirante comprenant un halogénure basique d'aluminium en poudre en suspension dans un véhicule dans lequel le rapport molaire aluminium: halogénure dans l'halogénure basique d'aluminium est de 1,7:1 à 2,2:1 et au moins 20% de l'aluminium est contenu sous forme du composé $Al_{13}O_{40}$.